Europäisches Patentamt

European Patent Office

Office européen des brevets

(11) Numéro de publication : **0 466 558 A1**

(12)

# DEMANDE DE BREVET EUROPEEN

(21) Numéro de dépôt : 91401830.4

(22) Date de dépôt : 03.07.91

(51) Int. Cl.[5] : **C07C 15/107,** C07C 2/66, C11D 1/22

(30) Priorité : **09.07.90 FR 9008801**

(43) Date de publication de la demande :
**15.01.92 Bulletin 92/03**

(84) Etats contractants désignés :
**AT BE DE ES GB IT NL**

(71) Demandeur : **INSTITUT FRANCAIS DU PETROLE**
**4, avenue de Bois-Préau**
**F-92502 Rueil-Malmaison (FR)**

(72) Inventeur : **Juguin, Bernard**
**décédé**
**- (FR)**
Inventeur : **Raatz, Francis**
**25, rue de la Carrière**
**F-57000 Saint Avold (FR)**
Inventeur : **Travers, Christine**
**25bis, rue des Coudreaux**
**F-92500 Rueil Malmaison (FR)**
Inventeur : **Martino, Germain**
**Bât. Condé 80, avenue F. Lefebvre**
**F-78300 Poissy (FR)**

(54) Procédé de production de 2- et 3-phenylalcanes utilisant un catalyseur à base de mordénite modifiée.

(57)   Procédé de production d'un mélange de 2-phénylalcanes et 3-phénylalcanes dans lequel on fait réagir du benzène avec une charge renfermant au moins une oléfine linéaire comprenant de 9 à 16 atomes de carbone dans sa molécule au contact d'un catalyseur à base d'une mordénite désaluminée ayant un rapport atomique Si/Al global compris entre 15 et 85, une teneur pondérale en sodium inférieure à 1000 ppm, une proportion d'espèces aluminiques extra-réseau très faible ou nulle, un volume de maille élémentaire inférieur à 2,760 nm³, une fréquence de vibration asymétrique des tétraèdres $TO_4$ (T = Al ou Si) supérieure à 1076 cm$^{-1}$ dans le spectre infra-rouge et un volume microporeux, mesuré par adsorption d'azote à 77K sous une pression partielle d'azote de 0,19, supérieur à 0,150 cm³ (liquide)/g. Utilisation du mélange de 2- et 3- phénylalcanes obtenu comme base pour détergents biodégradables.

EP 0 466 558 A1

La présente invention concerne un procédé de production de 2-phénylalcanes et de 3-phénylalcanes par alkylation du benzène au moyen d'oléfine(s) linéaire(s) comprenant de 9 à 16 atomes de carbone, plus particulièrement de 10 à 14 atomes de carbone, en présence d'au moins un catalyseur à base d'une mordénite modifiée.

Les 2- et 3- phénylalcanes obtenus selon l'invention constituent des composés pour la formulation, après sulfonation, de détergents biodégradables.

Actuellement, les bases pour détergents biodégradables font largement appel aux alkylbenzènes linéaires. La production de ce type de composés est en croissance régulière : elle avoisine 650 000 tonnes/an à l'échelle de l'Europe de l'Ouest par exemple. Une des propriétés principales recherchée pour ces composés, après l'étape de sulfonation, est, outre leur pouvoir détergent, leur biodégradabilité. Pour assurer une biodégradabilité maximale, le groupement alkyl doit être linéaire et long, la distance entre le groupe sulfonate et le carbone terminal de la chaine linéaire doit être maximal, le groupe phényl doit être le plus près possible d'une des extrémités du groupe alkyl. Les alkylbenzènes linéaires du type 2- et 3- phénylalcanes sont ainsi les plus appropriés pour atteindre cet objectif, les agents d'alkylation du benzène les plus intéressants étant constitués par les oléfines linéaires en $C_9$-$C_{16}$, de préférence en $C_{10}$-$C_{14}$.

Les alkylbenzènes linéaires obtenus par alkylation du benzène au moyen d'oléfine(s) linéaire(s) sont préparés aujourd'hui par deux grands procédés.

Le premier procédé est licencé par UOP (B. Vora, P. Pujado, J. Spinner, T. Imai, Hydrocarbon Processing, Nov. 1984, p.86 ; Petrochemical Handbook, Hydrocarbon Processing, Nov. 1987, p.63). Ce procédé utilise, lors de l'étape d'alkylation du benzène, de l'acide fluorhydrique comme agent catalytique.

Le second procédé est licencé par ARCO Technology Inc (Petrochemical Handbook, Nov. 1985, p.127). Le catalyseur d'alkylation est ici de type Friedel-Craft, en particulier à base de AlCl3.

Ces deux procédés présentent plusieurs inconvénients majeurs. Un des inconvénients résulte de la non-sélectivité des catalyseurs utilisés dans ces procédés. En effet, si ces catalyseurs sont très sélectifs du point de vue alkylation, ils conduisent à la formation de l'ensemble des phénylalcanes linéaires possibles. En plus de 2- et 3- phénylalcanes, il y a formation de 4-, 5- et 6- phénylalcanes en raison de l'isomérisation de position de la double liaison de l'oléfine initiale. Un autre inconvénient a trait à des contraintes d'environnement. Le procédé UOP basé sur l'utilisation d'acide fluorhydrique pose des problèmes de sécurité sévères d'une part et de retraitement des déchets d'autre part. Le procédé ARCO pose le problème classique des procédés basés sur des catalyseurs Friedel-Craft, en l'occurence le problème des rejets ; en effet, pour ce type de procédé, il est nécessaire de neutraliser les effluents par une solution basique en sortie de réacteur. A ces divers inconvénients s'ajoutent pour les deux procédés les difficultés liées à la séparation du catalyseur des produits de réaction.

Ces diverses contraintes expliquent l'intérêt qu'il y aurait à mettre au point un procédé d'alkylation du benzène par les oléfines linéaires en présence d'un catalyseur solide possédant des propriétés de sélectivité géométrique conduisant à une sélectivité améliorée en 2-phénylalcanes et 3-phénylalcanes. Une telle formule catalytique permettrait, d'une part, d'éliminer les problèmes d'environnement et de séparation catalyseur-effluent, et, d'autre part, de maximiser le production des 2- et 3-phénylalcanes qui sont les produits les plus recherchés. De tels catalyseurs solides à sélectivité géométrique pourraient être constitués par certaines zéolithes. Ainsi, l'utilisation de ce type de solides pour l'alkylation du benzène a déjà été décrite à plusieurs reprises. Bowes (brevet US-A-3.716.596) a revendiqué l'utilisation d'une zéolithe H-ZSM4 de rapport molaire $SiO_2$/$Al_2O_3$ de 7,7) dès 1973. Plus récemment, Young (brevet US-A-4.301.317) a revendiqué toute une série de zéolithes pour l'alkylation du benzène par les oléfines linéaires longues. Parmi les structures citées par cet auteur, on trouve notamment : la cancrinite, la gmelinite, la mordénite, l'offrétite et la ZSM12. Avant utilisation, ces zéolithes doivent être transformées au moins partiellement en une forme hydrogène. La technique requise pour procéder à cette transformation consiste à remplacer les cations initialement présents dans le solide par des précurseurs de protons, tels que les ions ammonium, puis à calciner en présence de vapeur d'eau la forme ammonium ainsi obtenue. Le produit résultant de cette transformation initiale peut alors être soumis à divers traitements chimiques, en particulier des traitements de désalumination. Les zéolithes préparées selon cette technique (US-A-4.301317) se révèlent assez performantes pour l'alkylation du benzène par les oléfines linéaires comprenant au moins 5 atomes de carbone. La température de réaction requise doit être supérieure à 50°C et la pression supérieure à 1 MPa.

La demanderesse a découvert que certaines mordénites ayant des propriétés physico-chimiques bien spécifiques (voir ci-dessous), par exemple l'absence d'espèces extra-réseau, notamment d'espèces aluminiques extra-réseau, présentent, en alkylation du benzène au moyen d'oléfine(s) linéaire(s) comprenant de 9 à 16 atomes de carbone, plus particulièrement de 10 à 14 atomes de carbone, des performances catalytiques supérieures à celles des catalyseurs décrits dans l'art antérieur ; elles sont notamment à la fois très actives, très sélectives et surtout plus résistantes à la désactivation.

3

Le procédé de production d'un mélange de 2- et 3- phénylalcanes selon l'invention consiste à faire réagir, dans une zone de réaction, du benzène avec une charge renfermant au moins une oléfine linéaire comprenant de 9 à 16 atomes de carbone, de préférence de 10 à 14 atomes de carbone, dans sa molécule au contact d'au moins un catalyseur à base d'une mordénite désaluminée de rapport atomique Si/Al global compris entre 15 et 85, de préférence entre 15 et 60 et de manière encore plus préférée entre 20 et 50, ladite mordénite désaluminée présentant en outre les caractéristiques suivantes :

– une teneur pondérale en sodium inférieure à 1000 ppm, de préférence inférieure à 250 ppm (soit un rapport atomique Na/Al inférieur à 0,75, de préférence inférieur à 0,25) ;

– une proportion d'espèces aluminiques extra-réseau très faible ou nulle ; sa teneur en espèces aluminiques extra-réseau est telle qu'on ne détecte pas de signal attribuable à de telles espèces, soit par RMN de $^{27}$Al en utilisant la technique de la rotation à l'angle magique, soit par spectroscopie infra-rouge dans la région des groupes hydroxyles ; de manière plus quantitative, le rapport de l'intensité des signaux correspondant aux espèces aluminiques hors charpente (extra-réseau) sur l'intensité des signaux correspondant aux espèces aluminiques de charpente est inférieur à 0,15, de préférence inférieur à 0,05, pour les deux techniques de caractérisation ;

– un volume de maille élémentaire inférieur à 2,760 nm$^3$ (1 nm = $10^{-9}$ m), de préférence inférieur à 2,745 nm$^3$ ;

– une fréquence de vibration asymétrique des tétraèdres T0$_4$ (T = Al ou Si) supérieure à 1076 cm$^{-1}$, de préférence supérieure à 1082 cm$^{-1}$, dans le spectre infra-rouge ;

– un volume microporeux, mesuré par adsorption d'azote à 77K sous une pression partielle d'azote de 0,19, supérieur à 0,150 cm$^3$ (liquide)/g, de préférence supérieur à 0,190 cm$^3$ (liquide)/g.

Ladite mordénite présente généralement, du fait d'un traitement de désalumination particulier permettant de réduire considérablement sa teneur en espèces aluminiques extra-réseau, un réseau poreux secondaire (ou réseau mésoporeux) dont le volume déterminé selon la théorie de DUBININ est supérieur 0,020 cm$^3$/g, de préférence supérieur à 0,040 cm$^3$/g ; des détails sur cette technique de détermination du volume mésoporeux sont donnés par F. RAATZ et H. AJOT dans Stud. Surf. Sci. Cat., vol. 39, p. 119,1988.

Les mordénites employées dans la présente invention sont fabriquées à partir de mordénites de base d'origine naturelle ou synthétique. On utilise de préférence comme mordénites de base des mordénites synthétiques, car elles peuvent être préparées en phase très pure avec un rapport atomique Si/Al global contrôlé variant entre 5 et 20 environ. Par ailleurs, ces mordénites synthétiques sont exemptes d'impuretés métalliques préjudiciables au niveau des performances catalytiques. De manière préférée, on emploie comme mordénites de base des mordénites synthétiques ayant après synthèse un rapport atomique Si/Al global inférieur à environ 10, ce qui permet notamment, après un traitement de désalumination, de développer un réseau mésoporeux important au sein des cristaux de la mordénite finale. Parmi ces mordénites synthétiques de rapport atomique Si/Al global inférieur à environ 10, on peut éventuellement choisir la mordénite de type "petits pores" dont la synthèse est notamment décrite par D. DOMINE et J. QUOBEX dans Molecular Sieves, Society of Chemical Industry, London, 1978, p. 78. Cette mordénite de par ses propriétés structurales particulières peut permettre d'améliorer davantage encore la sélectivité en 2- et 3- phénylalcanes de la mordénite finale.

Pour obtenir les mordénites ayant les caractéristiques mentionnées précédemment, les mordénites de base doivent subir un traitement de désalumination spécifique éventuellement précédé d'une étape d'échange ionique.

L'étape d'échange ionique optionnelle consiste généralement en au moins un traitement dans des solutions de sel d'ammonium (nitrate, sulfate, oxalate etc) de concentration en ammonium comprise entre 0,01 et ION, à une température comprise entre 10 et 180°C (échange sous pression autogène éventuellement), pendant une durée supérieure à 10 minutes environ.

Le traitement de désalumination est une attaque acide qui consiste en au moins un traitement dans des solutions d'acide de nature diverse (HCl, HN0$_3$, H$_2$S0$_4$, HF etc), a des températures comprises entre 10 et 150°C (attaque sous pression autogène éventuellement). Les concentrations en acide sont comprises de préférence entre 1,5 et 7N. Les rapports volumes de solution sur poids de solide sec sont compris entre 3 et 20 cm$^3$/g et, de manière avantageuse, entre 3,5 et 7 cm$^3$/g. La durée du traitement est d'au moins 10 minutes. Pour atteindre les spécifications désirées, on pourra avoir recours à un nombre limité d'attaques acides réalisées dans des conditions sévères ou à un nombre plus important d'attaques acides réalisées dans des conditions modérées.

Par rapport aux techniques de préparation décrites dans l'art antérieur, la voie de préparation utilisée pour l'invention, outre sa plus grande simplicité, permet d'obtenir des solides véritablement exempts d'espèces extra-réseau et dont le volume mésoporeux est libre de tous débris aluminiques. En effet, il est bien connu dans la littérature scientifique que le recours à des calcinations des zéolithes sous forme ammonium ou hydrogène, et plus particulièrement le recours à des calcinations sous vapeur d'eau, conduit à la formation d'espèces aluminiques extra-réseau qu'il est difficile sinon impossible d'extraire complètement par des traitements chimiques ultérieurs. Ces espèces extra-réseau, en particulier les espèces extra-réseau de nature aluminique, sont connues pour être des poisons des sites acides de charpente et sont de plus des agents de blocage du réseau poreux secondaire éventuellement formé lors de l'étape de calcination sous vapeur d'eau.

Sans nous lier à une théorie particulière, il est probable que les performances supérieures des mordénites employées dans l'invention résultent au moins en partie de l'absence d'espèces extra-réseau, en particulier d'espèces aluminiques extra-réseau, les aluminium extraits de la charpente lors de l'attaque acide étant ensuite directement solubilisés en solution. Ceci permet l'obtention de solides d'acidité maximale et dont les propriétés de diffusion sont grandement améliorées : acidité de type Bronsted uniquement liée aux aluminium de charpente (pas d'empoisonnement des sites de charpente par les aluminium extra-réseau, pas de sites acides hors charpente), réseaux micro et mésoporeux complètement libres de tous débris.

En effet, comme cela sera illustré dans les exemples, les mordénites utilisées dans la présente invention permettent de réaliser l'alkylation du benzène par les oléfines linéaires à très faibles températures, en particulier à une température inférieure à 60°C et même à 20°C, ce qui correspond classiquement à la température ambiante. Par ailleurs, la durée de cycle avec les catalyseurs utilisés dans l'invention est nettement améliorée : elle est de l'ordre de plusieurs mois à comparer à plusieurs semaines avec des mordénites préparées par une voie du type calcination sous vapeur d'eau suivie d'attaque(s) acide(s).

La mordénite désaluminée définie précédemment peut être utilisée, dans la présente invention, seule ou en mélange avec un liant ou une matrice généralement choisis dans le groupe formé par les argiles, les alumines, la silice, la magnésie, la zircone, l'oxyde de titane, l'oxyde de bore et toute combinaison d'au moins deux des composés précités comme la silice-alumine, la silice-magnésie etc. Toutes les méthodes connues d'agglomération et de mise en forme sont applicables, telles que par exemple l'extrusion, la pastillage, la coagulation en goutte, le séchage par atomisation etc.

On utilise ainsi dans le procédé selon l'invention au moins un catalyseur à base d'une mordénite désaluminée ayant les caractéristiques définies précédemment, contenant généralement 1 à 100 %, de préférence 20 à 98 % et, par exemple, 40 à 98 % en poids de ladite mordénite désaluminée et 0 à 99 %, de préférence 2 à 80 % et, par exemple, 2 à 60 % en poids d'une matrice.

Selon une variante préférée du procédé de l'invention, on fait réagir, dans une zone de réaction, du benzène avec une charge renfermant au moins une oléfine linéaire comprenant de 9 à 16 atomes de carbone, de préférence de 10 à 14 atomes de carbones, dans sa molécule, au contact d'au moins un catalyseur à base d'une mordénite désaluminée ayant les caractéristiques définies précédemment (réaction d'alkylation), puis on fractionne le produit obtenu de manière à recueillir séparément une première fraction renfermant du benzène non-converti, une deuxième fraction renfermant au moins une oléfine linéaire $C_9$-$C_{16}$ (de préférence $C_{10}$-$C_{14}$) non-convertie, une troisième fraction renfermant des 2- et 3- phénylalcanes et une quatrième fraction renfermant au moins un poly-alkylbenzène (ou fraction poly-alkylbenzène), celle-ci étant ensuite, au moins en partie, recyclée vers ladite zone de réaction où elle réagit donc avec du benzène au contact dudit catalyseur, c'est-à-dire du catalyseur mentionné ci-dessus à base de la mordénite désaluminée définie précédemment, afin d'être au moins en partie transalkylée (réaction de transalkylation), et on recueille un mélange de 2- et 3- phénylalcanes.

Cette variante de l'invention est donc notamment caractérisée par le fait que les réactions d'alkylation et de transalkylation ont lieu conjointement dans la même zone de réaction (c'est-à-dire dans le même réacteur) en présence du même catalyseur.

De manière préférée, la première fraction renfermant du benzène non-converti à l'issue de la réaction d'alkylation est au moins en partie recyclée vers ladite zone de réaction : ainsi, au moins une partie du benzène réagissant avec au moins une partie de la quatrième fraction (ou fraction poly-alkylbenzène) recyclée est constituée de benzène non-converti lors de la réaction d'alkylation, donc de benzène non-converti provenant de ladite première fraction.

De même, de manière préférée, la deuxième fraction renfermant au moins une oléfine linéaire $C_9$-$C_{16}$ (habituellement $C_{10}$-$C_{14}$) non-convertie à l'issue de la réaction d'alkylation est au moins en partie recyclée vers ladite zone de réaction.

La partie recyclée de la quatrième fraction contient de préférence essentiellement au moins un dialkylbenzène et est de préférence sensiblement exempte d'alkyl-aromatiques lourds qui peuvent être éliminés par fractionnement éventuellement.

Le mélange de 2-phénylalcanes et 3-phénylalcanes obtenu selon l'invention est de manière avantageuse quasi-exempt ou sensiblement exempt de n-phénylalcanes, n étant un nombre entier supérieur ou égal à 4.

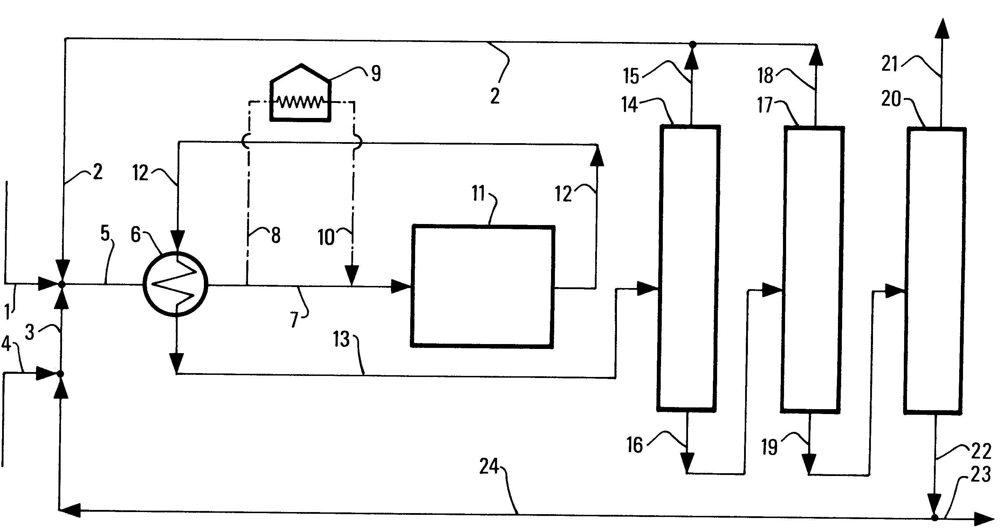

La figure unique illustre un mode particulier de réalisation de l'invention.

Du benzène frais (conduite 1) est mélangé, d'une part, avec un mélange (conduite 2) de benzène et d'oléfines linéaires $C_{10}$-$C_{14}$ (coupe dont l'intervalle de distillation se situe entre 165 et 240°C), le benzène provenant, par la conduite 15, de la tête d'une première colonne de fractionnement 14 et lesdites oléfines linéaires $C_{10}$-$C_{14}$

provenant, par la conduite 18, de la tête d'une deuxième colonne de fractionnement 17, et, d'autre part, avec un mélange (conduite 3) d'oléfines linéaires "fraîches" $C_{10}$-$C_{14}$ arrivant par la conduite 4 et de dialkylbenzènes provenant, par la conduite 24, du fond (conduite 22) d'une troisième colonne de fractionnement 20, après une purge éventuelle (conduite 23). Le mélange global (ou charge) obtenu est ensuite envoyé, par la conduite 5, dans l'échangeur de chaleur 6 où il est préchauffé par échange de chaleur avec l'effluent issu, par la conduite 12, du réacteur d'alkylation 11. Puis le mélange est envoyé, après son séjour dans l'échangeur de chaleur 6, dans le réacteur d'alkylation 11 par la conduite 7. Eventuellement, si le préchauffage dans l'échangeur de chaleur 6 est insuffisant, le mélange sortant de cet échangeur 6 est préalablement envoyé, par la conduite 8, dans le four de chauffage 9, d'où il ressort par la conduite 10 pour se diriger vers le réacteur d'alkylation 11. A la sortie du réacteur 11, l'effluent est envoyé, par la conduite 12, vers l'échangeur de chaleur 6, puis, par la conduite 13, vers une première colonne de fractionnement 14. En tête de cette première colonne de fractionnement 14, on sort, par la conduite 15, le benzène en excès qui n'a pas réagi et que l'on recycle ensuite, après mélange avec les oléfines linéaires $C_{10}$-$C_{14}$ non-transformées provenant, par la conduite 18, de la tête d'une deuxième colonne de fractionnement 17, vers l'entrée du réacteur 11 par la conduite 2. En fond de cette première colonne de fractionnement 14, on recueille un mélange qui est envoyé par la conduite 16, vers une deuxième colonne de fractionnement 17. En tête de cette deuxième colonne de fractionnement 17, on recueille, par la conduite 18, les oléfines linéaires $C_{10}$-$C_{14}$ non-transformées que l'on recycle ensuite, après mélange avec le benzène provenant, par la conduite 15, de la tête de la première colonne de fractionnement 14, vers l'entrée du réacteur 11 par la conduite 2. En fond de cette deuxième colonne de fractionnement 17, on soutire un mélange qui est envoyé, par la conduite 19, vers une troisième colonne de fractionnement 20. En tête de cette troisième colonne de fractionnement 20, on recueille, par la conduite 21, un mélange de 2-phénylalcanes et 3-phénylalcanes (coupe dont l'intervalle de distillation est situé entre 290 et 370°C) qui est envoyé au stockage. En fond de cette troisième colonne de fractionnement 20, on soutire, par la conduite 22, des dialkylbenzènes dont au moins une grande partie est recyclée, par la conduite 24, vers le réacteur 11 et dont une faible quantité est éventuellement purgée du circuit par la conduite 23.

Le procédé selon la présente invention peut être effectué à une température inférieure à 300°C, par exemple inférieure à 60°C, sous une pression de 1 à 10 MPa, avec un débit d'hydrocarbures liquides (vitesse spatiale) d'environ 0,5 à 50 volumes par volume de catalyseur et par heure et avec un rapport molaire benzène/(oléfine(s) $C_9$-$C_{16}$) compris entre 1 et 20.

Les exemples suivants illustrent l'invention sans toutefois en limiter la portée.

EXEMPLE 1 : Préparation de trois catalyseurs A, B, C.

La matière première utilisée pour préparer ces divers catalyseurs est une mordénite larges pores de la Société Tosoh, sous forme sodique, référencée TSZ600NAA. Sa formule chimique, à l'état anhydre, est Na, $AlO_2(SiO_2)_{5,1}$ et sa teneur en sodium est de l'ordre de 5 % en poids.

100 grammes de cette poudre sont portés à reflux à 100°C pendant 2 heures dans une solution de nitrate d'ammmonium 4M ; le volume de la solution de nitrate d'ammonium engagée est égal à 4 fois le poids de mordénite sèche (V/P = 4 cm³/g). Cette opération d'échange cationique est recommencée 3 fois. La teneur pondérale en sodium du produit obtenu est d'environ 500 ppm.

Ce produit est ensuite séparé en trois partie égales Ao, Bo et Co qui sont chacune soumises à une attaque acide à l'aide de solutions aqueuses d'acide nitrique respectivement de normalité 0,9 N, 4,5 N et 8 N : chaque solide est ainsi porté à reflux dans la solution aqueuse d'acide nitrique, avec un rapport V/P égal à 4 cm³/g, puis lavé à l'eau.

Les caractéristiques des mordénites obtenues Ao, Bo et Co figurent dans le tableau 1.

## TABLEAU 1

|  | Ao | Bo | Co |
|---|---|---|---|
| Na (ppm poids) | 185 | 25 | 10 |
| Si/Al (atomique global) | 13 | 40 | 90 |
| Volume de maille élémentaire $(nm^3)$ | 2,747 | 2,715 | 2,690 |
| Fréquence infra-rouge $TO_4$ $(cm^{-1})$ | 1080,2 | 1086,0 | 1091,7 |
| Volume microporeux $(cm^3/g)$ (adsorption d'azote) | 0,260 | 0,272 | 0,259 |
| Volume mésoporeux $(cm^3/g)$ (Dubinin) | 0,185 | 0,178 | 0,175 |

La caractérisation de ces solides par RMN de $^{27}$Al haute-résolution avec un spectromètre Brucker 400 MHz et par infra-rouge dans la région des groupes hydroxyles avec un spectromètre à transformée de Fourrier montre qu'ils sont exempts d'espèces aluminiques extra-réseau.

Chaque mordénite Ao, Bo et Co préparée précédemment est ensuite mélangée avec du gel d'alumine. Chaque mélange obtenu est ensuite mis sous forme d'extrudés de diamètre égal à environ 1,8 mm par passage au travers d'une filière. Les extrudés sont ensuite séchés dans une étuve à 120°C pendant une nuit, puis calcinés sous air sec jusqu'à 550°C environ.

On obtient alors des catalyseurs A, B et C à base respectivement des mordénites Ao, Bo et Co, contenant 80 % en poids de mordénite et 20 % en poids d'alumine.

## EXEMPLE 2

Les trois catalyseurs A, B et C, préparés dans l'exemple 1, sont chacun testés en alkylation du benzène par le dodécène-1, pour la production de 2- et 3-phényldodécanes, dans les conditions opératoires suivantes :
- température : 50°C
- pression : 4 MPa
- débit horaire de charge liquide égal à 3 fois le volume du catalyseur
- rapport molaire benzène/dodécène-1 : 5,5.

Les résultats obtenus sont présentés dans le tableau 2.

On peut ainsi constater qu'il est préférable de travailler avec les catalyseurs préconisés par l'invention, c'est-à-dire qu'il faut utiliser des mordénites désaluminées ayant notamment un rapport atomique Si/Al global compris entre 15 et 85, de préférence entre 15 et 60. En effet, le catalyseur à base de mordénite ayant un rapport atomique Si/Al global inférieur à 15 (cas du catalyseur A) est actif mais très peu sélectif ; de plus, il provoque, d'une part, une réaction d'isomérisation du dodécène-1, et, d'autre part, une formation non négligeable de 4-, 5- et 6- phényldodécanes qui conduisent à des détergents dont la biodégradabilité est faible. Le catalyseur à base de mordénite ayant un rapport atomique Si/Al global supérieur à 85 (cas du catalyseur C) est moins actif et n'a pas une sélectivité très satisfaisante en 2- et 3- phényldodécanes.

## TABLEAU 2

| CATALYSEUR | A | B | C |
|---|---|---|---|
| COMPOSITION DE LA CHARGE (% poids) | | | |
| Benzène | 71,7 | 71,7 | 71,7 |
| Dodécène-1 | 28,3 | 28,3 | 28,3 |
| COMPOSITION DU PRODUIT OBTENU (% poids) | | | |
| Benzène | 62,6 | 59,4 | 61,3 |
| Dodécène-1 | - | - | 3,1 |
| Isomères du dodécène-1 | 7,9 | - | - |
| 2-phényldodécane | 14,4 | 31,4 | 26,6 |
| 3-phényldodécane | 8,6 | 4,4 | 3,3 |
| 4-phényldodécane | 2,7 | 0,4 | 0,4 |
| 5-phényldodécane | 1,3 | - | - |
| 6-phényldodécane | 0,5 | - | - |
| Didodécylbenzènes | 1,8 | 4,1 | 4,4 |
| Résidu lourd | 0,2 | 0,3 | 0,9 |
| TAUX DE CONVERSION PAR PASSE | | | |
| Benzène | 12,7 % | 17,2 % | 14,5 % |
| Dodécène-1 | 100 % | 100 % | 89 % |
| SELECTIVITES PAR RAPPORT AU DODECENE-1 TRANSFORME | | | |
| 2-phényldodécane | 34,7 % | 75,8 % | 72,1 % |
| 3-phényldodécane | 20,8 % | 10,6 % | 8,9 % |
| Total | 55,5 % | 86,4 % | 81 % |

## EXEMPLE 3

En opérant selon le procédé de l'invention conforme à la figure unique, c'est-à-dire notamment en recyclant les didodécylbenzènes et le benzène vers le réacteur d'alkylation contenant le catalyseur B préparé dans

EP 0 466 558 A1

l'exemple 1, on obtient une sélectivité totale en 2- et 3-phényldodécanes par rapport au dodécène-1 transformé de 96,2 %. Ainsi, on obtient donc, avec une sélectivité très élevée, un mélange de 2- et 3-phényldodécanes conduisant à des détergents de très grande qualité et parfaitement biodégradables.

EXEMPLE 4 : Préparation d'un catalyseur D.

La matière première utilisée ici est une mordénite petits pores de la Société Chimique de la Grande Paroisse, référencée Alite 150. Sa formule chimique, à l'état anhydre, est $Na,AlO_2(SiO_2)_{5,5}$ et sa teneur en sodium est de 5,3 % en poids.

50 grammes de cette poudre sont portés à reflux à 100°C pendant 2 heures dans une solution de nitrate d'ammonium 4M avec un rapport V/P égal à 4 cm$^3$/g. Cette opération d'échange cationique est recommencée 3 fois. La teneur pondérale en sodium du produit obtenu est d'environ 700 ppm.

Ce produit est ensuite soumis à quatre attaques acides consécutives à l'aide de solutions aqueuses d'acide nitrique 6N : pour chaque attaque acide, le produit est ainsi porté à reflux dans la solution aqueuse d'acide nitrique pendant 2 heures, avec un rapport V/P égal à 4 cm$^3$/g. Après chaque attaque acide, le produit est lavé à l'eau.

La mordénite obtenue Do possède une teneur pondérale en sodium inférieure à 10 ppm, un rapport atomique Si/Al global de 10,7, un volume de maille élémentaire de 2,750 nm$^3$, une fréquence asymétrique des tétraèdres $TO_4$ de 1076,3 cm$^{-1}$ dans le spectre infra-rouge, un volume microporeux de 0,285 cm$^3$/g et un volume mésoporeux de 0,198 cm$^3$/g.

La caractérisation de ce solide par RMN de $^{27}Al$ et par infra-rouge dans la région des groupes hydroxyles (en utilisant les mêmes techniques que celles mentionnées dans l'exemple 1) montre qu'il est exempt d'espèces aluminiques extra-réseau.

Ce solide est ensuite mis en forme et calciné dans les mêmes conditions que celles décrites dans l'exemple 1.

On obtient alors un catalyseur D à base de la mordénite Do, contenant 80 % en poids de ladite mordénite et 20 % en poids d'alumine.

EXEMPLE 5

Le catalyseur D, préparé dans l'exemple 4, est testé en alkylation du benzène par le dodécène-1, pour la production de 2- et 3-phényldodécanes, dans des conditions opératoires identiques à celles utilisées dans l'exemple 2.

Les résultats obtenus sont présentés dans le tableau 3.

On constate que le catalyseur D, non conforme à ceux préconisés par l'invention, est actif mais très peu sélectif ; il provoque, d'une part, une réaction d'isomérisation du dodécène-1, et, d'autre part, une formation assez importante de 4-, 5- et 6- phényldodécanes.

EXEMPLE 6 : Préparation d'un catalyseur E.

A partir de la mordénite larges pores de la Société Tosoh, échangée au nitrate d'ammonium par la technique décrite dans l'exemple 1, on prépare une mordénite Eo par une succession de traitements steaming ou calcination en atmosphère confinée et attaque acide ; le produit Eo est préparé par steaming à 530°C de la mordénite sous forme ammonium pendant 2 heures, suivi d'une attaque acide par une solution aqueuse d'acide nitrique 1, 8N pendant 2 heures à la température de reflux.

9

## TABLEAU 3

| CATALYSEUR | D |
|---|---|
| COMPOSITION DE LA CHARGE (% poids) | |
| Benzène | 71,7 |
| Dodécène-1 | 28,3 |
| COMPOSITION DU PRODUIT OBTENU (% poids) | |
| Benzène | 62,7 |
| Dodécène-1 | - |
| Isomères du dodécène-1 | 8,5 |
| 2-phényldodécane | 13,1 |
| 3-phényldodécane | 9,2 |
| 4-phényldodécane | 3 |
| 5-phényldodécane | 1,4 |
| 6-phényldodécane | 0,5 |
| Didodécylbenzènes | 1,5 |
| Résidu lourd | 0,1 |
| TAUX DE CONVERSION PAR PASSE | |
| Benzène | 12,6 % |
| Dodécène-1 | 100 % |
| SELECTIVITES PAR RAPPORT AU DODECENE 1 TRANSFORME | |
| 2-phényldodécane | 31,7 % |
| 3-phényldodécane | 22,1 % |
| Total | 53,8 % |

La mordénite obtenue Eo possède une teneur pondérale en sodium de 110 ppm, un rapport atomique Si/Al global de 28, un volume de maille élémentaire de 2,725 nm³, une fréquence asymétrique des tétraèdres $TO_4$ de 1089,6 cm$^{-1}$ dans le spectre infra-rouge, un volume microporeux de 0,265 cm³/g et un volume mésoporeux de 0,182 cm³/g.

La caractérisation de ce solide par RMN de $^{27}$Al et par infra-rouge dans la région des groupes hydroxyles (en utilisant les mêmes techniques que celles mentionnées dans l'exemple 1 ) montre qu'il renferme une quantité importante d'espèces aluminiques extra-réseau.

Ce solide est ensuite mis en forme et calciné dans les mêmes conditions que celles décrites dans l'exemple 1.

On obtient alors un catalyseur E à base de la mordénite Eo, contenant 80 % en poids de ladite mordénite et 20 % en poids d'alumine.

## EXEMPLE 7

Le catalyseur E, préparé dans l'exemple 6, est testé en alkylation du benzène par le dodécène-1, pour la production de 2- et 3-phényldodécanes, dans des conditions opératoires identiques à celles utilisées dans l'exemple 2 (en particulier avec une charge de même composition).

Les taux de conversion par passe obtenus sont de 10,3 % pour le benzène et de 83 % pour le dodécène-1.

Les sélectivités par rapport au dodécène-1 transformé sont les suivantes :

$$
\begin{aligned}
&- \text{2-phényldodécane} : 25,3~\% \\
&- \text{3-phényldodécane} : \underline{16,2~\%} \\
&\qquad\qquad \text{Total} \qquad 41,5~\%
\end{aligned}
$$

On constate ainsi qu'il est très désavantageux de travailler avec une mordénite contenant une quantité importante d'espèces aluminiques extra-réseau.

## Revendications

1. Procédé de production d'un mélange de 2-phénylalcanes et 3-phénylalcanes dans lequel on fait réagir, dans une zone de réaction, du benzène avec une charge renfermant au moins une oléfine linéaire comprenant de 9 à 16 atomes de carbone dans sa molécule au contact d'au moins un catalyseur à base d'une mordénite désaluminée ayant un rapport atomique Si/Al global compris entre 15 et 85, une teneur pondérale en sodium inférieure à 1000 ppm, une proportion d'espèces aluminiques extra-réseau très faible ou nulle, un volume de maille élémentaire inférieur à 2,760 $nm^3$, une fréquence de vibration asymétrique des tétraèdres $T0_4$ (T = Al ou Si) supérieure à 1076 cm $^{-1}$ dans le spectre infra-rouge et un volume microporeux, mesuré par adsorption d'azote à 77K sous une pression partielle d'azote de 0,19, supérieur à 0,150 $cm^3$ (liquide)/g, dans lequel on fractionne ensuite le produit obtenu de manière à recueillir séparément une première fraction renfermant du benzène non-converti, une deuxième fraction renfermant au moins une oléfine linéaire, comprenant de 9 à 16 atomes de carbone dans sa molécule, non-convertie, une troisième fraction renfermant un mélange de 2-phénylalcanes et 3-phénylalcanes et une quatrième fraction renfermant au moins un poly-alkylbenzène, ladite quatrième fraction étant ensuite, au moins en partie, recyclée vers ladite zone de réaction où elle réagit avec du benzène en présence dudit catalyseur afin d'être au moins en partie transalkylée, et on recueille un mélange de 2-phénylalcanes et 3-phénylalcanes.

2. Procédé selon la revendication 1 dans lequel ladite mordénite désaluminée a un rapport atomique Si/Al global compris entre 15 et 60.

3. Procédé selon l'une des revendications 1 et 2 dans lequel ladite mordénite désaluminée est telle que le rapport de l'intensité des signaux correspondant aux espèces aluminiques hors charpente (extra-réseau) sur l'intensité des signaux correspondant aux espèces aluminiques de charpente (mesuré par RMN de $^{27}$Al et spectroscopie infra-rouge dans la région des groupes hydroxyles) est inférieur à 0,15.

4. Procédé selon l'une des revendications 1 à 3 dans lequel ladite mordénite désaluminée est telle que :
   – son rapport atomique Si/Al global est compris entre 20 et 50,
   – sa teneur pondérale en sodium est inférieure à 250 ppm,
   – le rapport de l'intensité des signaux correspondant aux espèces aluminiques hors charpente (extraréseau) sur l'intensité des signaux correpondant aux espèces aluminiques de charpente (mesuré par RMN de $^{27}$Al et spectroscopie infra-rouge dans la région des groupes hydroxyles) est inférieur à 0,05,
   – son volume de maille élémentaire est inférieur à 2,745 $nm^3$,

– la fréquence de vibration asymétrique des tétraèdres $TO_4$ (T = Al ou Si) est supérieur à 1082 cm$^{-1}$ dans le spectre infra-rouge,

– son volume microporeux, mesuré par adsorption d'azote à 77K sous une pression partielle d'azote de 0,19, est supérieur à 0,190 cm$^3$ (liquide)/g.

5. Procédé selon l'une des revendications 1 à 4 dans lequel ladite mordénite désaluminée présente un réseau poreux secondaire dont le volume est supérieur à 0,020 cm$^3$/g.

6. Procédé selon l'une des revendications 1 à 5 dans lequel ladite première fraction est au moins en partie recyclée vers ladite zone de réaction.

7. Procédé selon l'une des revendications 1 à 6 dans lequel ladite deuxième fraction est au moins en partie recyclée vers ladite zone de réaction.

8. Procédé selon l'une des revendications 1 à 7 dans lequel ledit catalyseur contient en outre une matrice choisie dans le groupe formé par les argiles, les alumines, la silice, la magnésie, la zircone, l'oxyde de titane, l'oxyde de bore et toute combinaison d'au moins deux des composés précités.

9. Procédé selon l'une des revendications 1 à 8 dans lequel la température opératoire est inférieure à 60°C.

10. Utilisation, après sulfonation, du mélange de 2-phénylalcanes et 3-phénylalcanes obtenu par le procédé selon l'une des revendications 1 à 9 comme base pour détergents biodégradables.

**Office européen des brevets**

## RAPPORT DE RECHERCHE EUROPEENNE

Numero de la demande

EP 91 40 1830

### DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | US-A-4 731 497  (GREY) --- | | C 07 C  15/107 C 07 C  2/66 C 11 D  1/22 |
| A | EP-A-0 366 515  (INSTITUT FRANCAIS DU PETROLE) --- | | |
| A | FR-A-2 084 704  (UNIVERSAL OIL PRODUCTS CO.) ----- | | |

**DOMAINES TECHNIQUES RECHERCHES (Int. Cl.5)**

C 07 C  2/00

Le présent rapport a été établi pour toutes les revendications

| Lieu de la recherche | Date d'achèvement de la recherche | Examinateur |
|---|---|---|
| LA HAYE | 30-09-1991 | VAN GEYT J.J.A. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)